# FASCICULE DE BREVET EUROPEEN

(11) **EP 1 711 162 B1**
(45) Date de publication et mention de la délivrance du brevet: **01.11.2017**
(21) Numéro de dépôt: 05726342.8
(22) Date de dépôt: 01.02.2005
(51) Int. Cl.: A61P 11/04, A61P 1/02, A61K 9/00, A61K 31/205, A61K 31/192, A61K 47/36, A61K 47/38, A61K 47/30, A61K 47/18

(54) **Composition pour l'utilisation dans le traitement local des affections bucco-pharyngées comprenant de l'ibuprofène ou du kétoprofène**
Zusammensetzung zur Verwendung bei der lokalen Behandlung von oropharyngealen Erkrankungen enthaltend Ibuprofen oder Ketoprofen
Composition for use in the local treatment of oropharyngeal conditions comprising ibuprofen or ketoprofen

(30) Priorité: 03.02.2004 FR 0450194
(43) Date de publication de la demande: 18.10.2006
(73) Titulaire: Perovitch, Philippe, 33950 Lege Cap Ferret (FR); Maury, Marc, 33160 Saint Medard en Jalles (FR)
(72) Inventeur: Perovitch, Philippe, 33950 Lege Cap Ferret (FR); Maury, Marc, 33160 Saint Medard en Jalles (FR)
(74) Mandataire: Riege, Christian
(86) Numéro de dépôt international: PCT/FR2005/050062
(87) Numéro de publication internationale: WO 2005/074885

(56) Documents cités:
- EP-A- 0 137 668
- EP-A- 0 159 604
- EP-A- 0 523 847
- EP-A- 0 935 961
- WO-A-92/00725
- WO-A-95/08988
- WO-A-95/15137
- WO-A-97/04808
- WO-A-97/20572
- WO-A-97/26866
- WO-A-98/52539
- WO-A-02/067916
- WO-A-02/083119
- WO-A-03/043600
- WO-A1-97/18802
- WO-A1-98/52540
- GB-A- 1 103 686

## Description

La présente invention concerne une composition mettant en oeuvre un procédé de diffusion à travers les muqueuses de la bouche et de la gorge de molécules insolubles en milieu aqueux, notamment pour le traitement des affections bucco pharyngées.

A titre d'exemple, les affections inflammatoires et douloureuses de la sphère bucco pharyngée sont pénalisantes pour les patients et il est nécessaire de constater que la pharmacopée est pauvre pour apporter un soulagement rapide, efficace, de durée suffisante et en limitant les effets secondaires.

Ces affections bucco pharyngées sont d'origine diverses et se développent dans la partie antérieure, sur les muqueuses du plancher et des parois de la bouche ou sur la partie postérieure, sur la muqueuse pharyngée.

La sphère bucco pharyngée étant une voie d'accès constante et privilégiée pour tous les germes et substances irritantes apportés par les voies aérienne et alimentaire. Cette zone muqueuse est aussi un lieu de développement privilégié de populations bactériennes, de virus plus ou moins pathogènes justifiant un traitement des inflammations qu'ils engendrent.

Ces inflammations peuvent être plus ou moins importantes et invalidantes allant de la simple sensation de gêne localisée à la présence de lésions macroscopiquement visibles du type de celles générées par les aphtoses.

De telles inflammations sont souvent dépourvues de signes cliniques majeurs comme de la fièvre ou des formations ganglionnaires.

Les traitements actuels prévoient l'utilisation de produits anti-inflammatoires et/ou antalgiques à administration locale : pulvérisations, pastilles à sucer, bains de bouche.

Quant aux médicaments disponibles ils sont extrêmement limités depuis la disparition de nombreux produits à titre de médicament, c'est-à-dire des compositions ayant une Autorisation de Mise sur le Marché.

C'est ainsi que les produits comportant des associations d'enzymes, lysozyme, papaïne, des anesthésiques de contact ou des antibiotiques locaux ont perdu leur AMM ou vont la perdre.

En effet, de tels produits comme les anesthésiants, en masquant la douleur plutôt qu'en traitant le mal dissimulaient la réalité de l'inflammation.

Une solution consiste à recourir à des anti-inflammatoires puissants qui permettent de réduire les douleurs tout en traitant aussi les inflammations associées.

De tels principes actifs sont administrés par la voie digestive avec tous les inconvénients associés.

Le principe actif doit donc être métabolisé par l'organisme induisant une distribution généralisée de la molécule dans l'ensemble des organes et tissus.

Cette large diffusion est inutile pour sa plus grande part puisque pour traiter les 2% représentant la sphère bucco pharyngée, on traite 100% de l'organisme.

De fait, il apparaît plusieurs problèmes à solutionner.

Le premier est qu'il faut administrer une dose suffisante au patient tenant compte de la dilution et de la dispersion dans l'organisme, pour que la partie significativement active qui atteint la zone affectée soit efficace.

Le deuxième est le temps de latence dû à la métabolisation et à la diffusion dans l'organisme avant que la molécule agisse et que le patient en ressente les bienfaits.

Le troisième résulte des conséquences qu'une telle diffusion massive de la molécule active peut provoquer dans l'organisme, conséquences qui se traduisent par des effets secondaires connus.

C'est ainsi que deux molécules connues l'ibuprofène et le kétoprofène, anti-inflammatoires très efficaces peuvent être utilisées pour traiter des affections sévères de la sphère bucco pharyngée mais ceci avec les conséquences exprimées ci-avant.

C'est ainsi que pour l'ibuprofène, on peut administrer des doses de 200 mg ou plus et pour le kétoprofène, on peut administrer des doses de 50 mg ou plus afin de tenir compte de la dilution dans l'organisme.

On sait aussi que le début de l'efficacité thérapeutique pour le patient intervient au plus tôt 45 min après la prise, correspondant au délai d'absorption digestive, de métabolisation et de diffusion tissulaire.

La concentration maximale du principe actif dans le sang est atteinte pour l'ibuprofène après 90 min et pour le kétoprofène au bout de 75 min sachant que leurs effets pharmacologiques respectifs sont d'une durée de 2 heures environ pour l'ibuprofène et de 1,5 heure pour le kétoprofène.

Quant aux effets secondaires, ils sont engendrés par la diffusion des molécules dans les compartiments vasculaires et tissulaires de l'organisme puisque ces molécules sont de nature lipophile leur conférant une forte biodisponibilité.

Ces effets se traduisent par des nausées, des vomissements, des gastralgies, des dyspepsies, des hémorragies occultes ou non, des troubles du transit des réactions d'hypersensibilité cutanées ou respiratoires, des vertiges ou céphalées ou encore des effets secondaires hépatiques, rénaux ou hématologiques.

On constate donc que les conséquences secondaires engendrées ou susceptibles de l'être par l'absorption de telles molécules sont sans commune mesure avec les douleurs et inflammations bucco pharyngées, fussent-elles invalidantes localement.

Les documents WO97/20572, WO98/52539, WO92/00725, WO98/5254 et WO97/18802 décrivent des compositions de l'état de la technique.

Le document WO97/20572 décrit une composition pharmaceutique pour application buccale comprenant un AINS et des céramides, sous forme de dentifrice, bain de bouche ou gel.

Le document WO98/52539 décrit un procédé de fabrication de pastilles de flurbiprofène racémique, utilisant une technique de sucres cuits.

Le document WO98/52540 décrit des compositions pharmaceutiques, sous forme de pastilles de sucres cuits ou de spray comprenant un AINS. Aucune forme de comprimé à sucer contenant du lysinate d'ibuprofène ou de kétoprofène n'est décrite.

Le document WO92/00725 décrit des compositions pharmaceutiques liquides.

Le document WO97/18802 décrit des compositions pharmaceutiques comprenant du flurbiprofène, sous forme de pastille de sucres cuits ou de spray. Aucune forme de comprimé à sucer contenant du lysiate d'ibuprofène ou de kétoprofène n'est décrit.

La composition selon la présente invention, donnée à titre d'exemple, a pour objet de pallier ces problèmes en agissant localement au niveau même des lésions et des inflammations avec des dosages très réduits, ne pouvant donc provoquer d'effets secondaires et en induisant un effet thérapeutique immédiat.

La présente invention a pour objet une composition selon la revendication 1. Des modes de réalisation avantageux sont décrits dans les revendications 2 à 4. La présente invention a aussi pour objet un comprimé selon les revendications 5 ou 6 et une utilisation selon la revendication 7.

La composition selon l'invention à base de dérivés de la famille des aryl-carboxyliques est maintenant décrite en détail en mettant en exergue les avantages induits et les solutions apportées aux problèmes posés par les thérapies actuellement proposées.

Il est à noter en préambule qu'il y a plusieurs forts préjugés à vaincre pour penser à administrer localement des dérivés de la famille des aryl-carboxyliques. En effet, ces composés actifs sont habituellement peu solubles dans l'eau et les milieux biologiques.

Ceci induit pour la mise en oeuvre de ce type de composition un procédé permettant deux effets, a priori antagonistes, la dissolution du principe actif malgré son caractère insoluble et sa diffusion active facilitée à travers les tissus muqueux grâce à son caractère lipophile.

De plus, le goût des composés retenus comme exemple de mise en oeuvre du procédé est totalement inacceptable, plus particulièrement l'ibuprofène, et interdit la mise en contact direct avec les organes et les muqueuses gustatives.

Il convient donc de recourir à des formulations galéniques qui assurent une diffusion lente, permettant l'usage local bucco-pharyngé et, grâce à leur parfaite dissolution, qui assurent un passage per-muqueux aisé du principe actif afin qu'il soit en mesure d'agir au niveau des lésions concernées.

Le procédé consiste à recourir à un sel de ces composés. En l'occurrence, la composition recourt à l'ibuprofène et au kétoprofène sous forme de lysinate résultant de la combinaison d'un amino-acide, la lysine, et des molécules d'ibuprofène et de kétoprofène.

Ces composés sont de type anti-inflammatoire et antalgiques périphériques. Ils agissent sur les médiateurs de l'inflammation, à savoir les enzymes tissulaires notamment les cyclo-oxygénases 1 et 2 et les prostaglandines.

Alors que les principes actifs ibuprofène et kétoprofène sont de nature lipophile, pratiquement insolubles dans l'eau, les formes de lysinates sont parfaitement hydrosolubles.

De fait, dans le cas de la composition selon la présente invention, il se produit dans un premier temps la solubilisation dans la salive buccale et dans un second temps la liaison au sel de lysine étant faible, la dissociation rapide survient et confère de nouveau aux principes actifs leur caractère lipophile.

Ce caractère lipophile leur permet alors de franchir aisément et passivement les membranes cellulaires muqueuses, elles aussi lipidiques puisqu'elles sont constituées de phospholipides.

Ayant pénétré, ces substances induisent un blocage des médiateurs tissulaires de l'inflammation.

Ensuite, afin de maintenir le ou les principes actifs étroitement en contact avec les muqueuses concernées, celles de la sphère bucco-pharyngée, il convient de formuler ces principes actifs avec au moins un agent combiné.

Ce premier agent a pour rôle, en plus de créer un film bioadhésif sur les muqueuses, de ralentir la dissolution et la libération du principe actif dans la salive et de le maintenir en place localement afin de limiter sa déperdition par déglutition.

De fait, ce premier agent est de type polymérique et assure simultanément à ces actions une dissolution au sein même de la matrice polymère.

Cet agent est choisi dans les familles suivantes :
1/ Dérivés cellulosiques :
   - carboxy-méthyl cellulose sodique,
   - hydroxy-éthyl cellulose,
   - hydroxy-propyl cellulose,
   - hydroxy-propyl méthyl cellulose ou promellose, ou
   - carboxy-méthyl cellulose.
2/ Gommes:
   - guar,
   - xanthane, ou
   - gomme arabique.
3/ Polymères :
   - acide alginique et dérivés,
   - polymère carboxy-vinylique,
   - carbomère,
   - macrogols,
   - polyéthylène glycol,
   - gélatine
   - povidone, ou
   - pectines.

Cet agent polymérique est intégré dans la forme galénique finale dans des proportions de l'ordre de 2 à 20%.

La forme galénique retenue pour l'administration de cette composition est un comprimé à sucer et à délitement lent.

A cet effet, un substrat préférentiel pour la fabrication d'un tel comprimé est un substrat soluble et très hydrophile. Par ces propriétés, sa seule présence provoque un flux osmotique en bouche qui facilite l'expression du principe actif dissous.

Ce substrat représente la grande majorité du comprimé fini.

Il est important lors de la fabrication de prévoir une très forte homogénéité de la distribution des principes actifs.

En effet, il faut éviter un apport localisé important dû à une concentration résultant d'une inhomogénéité de répartition car il convient que le principe actif ait été dissous dans la fraction polymérique grâce à l'eau qu'il absorbe par ses propriétés fortement hydrophiles et non dans la cavité buccale.

Comme les polymères des familles retenues sont très avides d'eau, les principes actifs ne sont pas mis au contact direct des muqueuses et il n'y a pas de résurgence du goût avant le passage per-muqueux.

De plus, une concentration trop rapide ou trop importante de principe actif libéré créerait un effet de saturation de la capacité d'absorption de la muqueuse et induirait simultanément des recristallisations des principes actifs et des ulcérations locales, ce qui est non nécessaire voire irritatif donc préjudiciable à une bonne administration.

L'association du substrat à l'agent polymérique permet d'éviter ces phénomènes de saturation et de s'opposer à la recristallisation des principes actifs en assurant une dissolution lente et une imprégnation régulière des principes actifs par la muqueuse. Cette association empêche également la déglutition et l'échappement par voir digestive des principes actifs.

Ainsi, l'effet conjugué du substrat et de l'agent polymérique associé exerce un double rôle de vecteur des principes actifs et de protecteur de la muqueuse contre les risques d'ulcérations des tissus mis au contact de ces mêmes principes actifs.

Un tel substrat sera choisi parmi la famille des carbohydrates :
- lactose
- glucose,
- saccharose,
- sorbitol,
- mannitol, et
- xylitol.

Une telle formulation galénique présente l'avantage de conférer à la composition une stabilité pharmaceutique dans le temps pour les principes actifs utilisés.

On peut citer deux exemples de formulations avec l'un et l'autre des deux principes actifs préférentiels retenus.

### Pour un comprimé de 1000mg :

- lysinate d'ibuprofène : 25 mg
- stéarate de magnésium : 10 mg
- talc : 50 mg
- aspartame : 15 mg
- métolose : 70 mg
- arôme : 20 mg
- sorbitol : 810 mg

### Pour un comprimé de 1000mg :

- lysinate de kétoprofène : 5 mg
- stéarate de magnésium : 10 mg
- talc : 50 mg
- aspartame : 15 mg
- métolose : 70 mg
- arôme : 20 mg
- sorbitol : 830 mg

Un comprimé avec une telle composition est réalisable industriellement, sans précaution draconienne, de la façon suivante :
- introduire à sec les constituants dans un mélangeur à retournement, avec démottage préalable sur une grille de 1 à 2 mm de maille,
- mélanger ces constituants pendant 10 à 20 minutes pour assurer une très bonne homogénéité de répartition,
- compresser le mélange obtenu sur une presse à comprimés pour obtenir un comprimé avec un délitement, lors de la prise par le patient de l'ordre de 1 à 5 minutes, et
- conditionnement sous blister ou en tubes étanches, connus en soi.

Un tel comprimé permet d'administrer une dose réduite de principe actif, d'obtenir une dissolution régulière, lente, complète en ménageant une rémanence buccale pour un effet immédiat et directement au contact des lésions inflammatoires.

Le procédé qui vient d'être décrit n'est pas limité à l'application qui vient d'être donnée à travers deux exemples mais il trouve application plus généralement pour des molécules lipophiles selon la revendication 1 apte à être administrées par voie sub-linguale per-muqueuse, plutôt à des doses inférieures à 100 mg. Ceci permet de conserver le bénéfice de gagner les différents territoires vasculaires et tissulaires avant de subir la première dégradation/métabolisation hépatique.

On peut citer comme molécules à usage local :
- des anti-inflammatoires : bufexamac, diclofénac, flurbiprofène, acide flufénamique, indométacine, acide méfénamique, naproxène, acide niflumique, sulindac, ténoxicam, ou
- des anti-mycosiques : éconazole, fenticonazole, miconazole.

On peut citer comme molécules aptes à bénéficier du procédé pour être administrées par voie sub-linguale :
- des antagilsques morphiniques à action centrale : fentanyl
- des anti-allergiques anti-nauséeux : diphenhydramine.

De tels composés sont difficilement solubles dans l'eau et le fait de les administrer par voie sub-linguale per-muqueuse sous forme de lysinate, permet une action rapide, efficace en supprimant la dégradation liée à la phase digestive et en diminuant fortement les doses administrées.

De plus, il est possible de les combiner pour le traitement simultané de plusieurs affections ou pour des actions complémentaires d'une même affection.

## Revendications

1. Composition pour l'utilisation dans le traitement local des affections bucco-pharyngées par diffusion à travers les tissus muqueux de molécules de nature lipophile, comprenant de l'ibuprofène ou du kétoprofène sous forme de lysinates de ces molécules, lesdits lysinates se solubilisant dans la salive buccale puis se dissociant en lysine et molécules pour redonner auxdites molécules leur caractère lipophile pour une absorption locale desdites molécules à travers les muqueuses de la bouche et de la gorge, ladite composition comprenant un substrat choisi parmi les carbohydrates, plus particulièrement le sorbitol ou le xylitol, **caractérisée en ce que** la composition est sous forme d'un comprimé à sucer et à délitement lent.

2. Composition pour l'utilisation selon la revendication 1, comprenant en outre un agent polymérique choisi parmi la famille des dérivés cellulosiques, plus particulièrement carboxy-méthyl cellulose sodique, hydroxy-éthyl cellulose, hydroxy-propyl cellulose, hydroxy-propyl méthyl cellulose ou promellose, ou carboxy-méthyl cellulose.

3. Composition pour l'utilisation selon la revendication 1, comprenant en outre un agent polymérique choisi parmi la famille des gommes telles que guar, gomme arabique ou xanthane.

4. Composition pour l'utilisation selon la revendication 1, comprenant en outre un agent polymérique choisi parmi les composés : acide alginique et dérivés, polymère carboxy-vinylique, carbomère, macrogols, polyéthylène glycols, gélatine, povidone ou pectines.

5. Comprimé incluant la composition selon la revendication 1 ou 2, **caractérisé en ce qu'**il comprend la formulation suivante :
- lysinate d' ibuprofène : 25 mg
- stéarate de magnésium : 10 mg
- talc : 50 mg
- aspartame : 15 mg
- métolose : 70 mg
- arôme : 20 mg
- sorbitol : 810 mg

6. Comprimé incluant la composition selon la revendication 1 ou 2, **caractérisé en ce qu'**il comprend la formulation suivante :
- lysinate de kétoprofène : 5 mg
- stéarate de magnésium : 10 mg
- talc : 50 mg
- aspartame : 15 mg
- métolose : 70 mg
- arôme : 20 mg
- sorbitol : 830 mg

7. Utilisation de la composition selon l'une quelconque des revendications 1 à 4, pour la fabrication d'un médicament destiné au traitement local des affections bucco pharyngées.

## Patentansprüche

1. Zusammensetzung zur Verwendung bei der lokalen Behandlung von Erkrankungen im Mund- und Rachenraum durch Diffusion von Molekülen lipophiler Natur über die Schleimhäute, wobei die Zusammensetzung Ibuprofen oder Ketoprofen in Form von Lysinaten dieser Moleküle enthält, wobei diese Lysinate im Mundspeichel solubilisiert werden und daraufhin wieder zu Lysin und Molekülen dissoziiert werden, um dadurch die lipophile Eigenschaft besagter Molekülen für eine lokale Absorption besagter Moleküle über die Schleimhäute in Mund und Rachen wiederherzugestellen, wobei besagte Zusammensetzung ein Substrat umfasst, das ausgewählt ist aus der Gruppe bestehend aus Kohlenhydraten, insbesondere Sorbitol oder Xylitol, **dadurch gekennzeichnet, dass** die Zusammensetzung in Form einer Lutschtablette mit langsamer Auflösung vorliegt.

2. Zusammensetzung zur Verwendung nach Anspruch 1, des Weiteren umfassend ein Polymeres, ausgewählt aus der Familie der Cellulosederivate, insbesondere Natriumcarboxymethylcellulose, Hydroxyethylcellulose, Hydroxypropylcellulose, Hydroxypropylmethylcellulose oder Promellose, oder Carboxymethylcellulose.

3. Zusammensetzung zur Verwendung nach Anspruch 1, des Weiteren umfassend ein Polymeres, ausgewählt aus der Familie der Gummis, beispielsweise Guar-Gummi, Gummiarabikum oder Xanthan-Gummi.

4. Zusammensetzung zur Verwendung nach Anspruch 1, des Weiteren umfassend ein Polymeres, ausgewählt aus folgenden Verbindungen: Alginsäure und -derivaten, Carboxyvinylpolymer, Carbomer, Macrogolen, Polyethylenglykolen, Gelatine, Povidon oder Pektinen.

5. Tablette mit der Zusammensetzung nach Anspruch 1 oder 2, **dadurch gekennzeichnet, dass** sie folgende Formulierung umfasst:
- Ibuprofenlysinat: 25 mg
- Magnesiumstearat: 10 mg
- Talk: 50 mg
- Aspartam: 15 mg
- Metolose: 70 mg
- Aromastoff: 20 mg
- Sorbitol: 810 mg

6. Tablette mit der Zusammensetzung nach Anspruch 1 oder 2, **dadurch gekennzeichnet, dass** sie folgende Formulierung umfasst:
- Ketoprofenlysinat: 5 mg
- Magnesiumstearat: 10 mg
- Talk: 50 mg
- Aspartam: 15 mg
- Metolose: 70 mg
- Aromastoff: 20 mg
- Sorbitol: 830 mg

7. Verwendung der Zusammensetzung nach einem der vorhergehenden Ansprüche 1 bis 4 für die Herstellung eines Medikaments zur lokalen Behandlung von Erkrankungen im Mund- und Rachenraum.

## Claims

1. Composition for use in the local treatment of buccopharyngeal ailments by diffusion through the mucous tissues of lipophilic molecules comprising ibuprofen or ketoprofen in the lysinate form of these molecules, said lysinates solubilizing in the oral saliva and then getting dissociated in lysine and molecules to give again to said molecules their lipophilic feature for a local absorption of said molecules through the mucous of the mouth and the throat, said composition comprising a substrate that is selected from among the carbohydrates, more particularly sorbitol or xylitol, **characterized in that** said composition is in form of a suckable slow-dissolving tablet.

2. Composition for use according to claim 1, further comprising a polymer agent that is selected from among the family of cellulose derivatives, more particularly carboxy-methyl cellulose that contains soda, hydroxy-ethyl cellulose, hydroxy-propyl cellulose, hydroxy-propyl methyl cellulose or promellose, or carboxy-methyl cellulose.

3. Composition for use according to claim 1, further comprising a polymer agent that is selected from among the family of gums, such as guar gum, gum Arabic, or xanthane gum.

4. Composition for use according to claim 1, further comprising a polymer agent that is selected from among the compounds: alginic acid and derivatives, carboxy-vinyl polymer, carbomer, macrogols, polyethylene glycols, gelatin, povidone, or pectins.

5. Tablet including the composition according to claim 1 or claim 2, **characterized in that** it comprises the following formulation:
- ibuprofen lysinate: 25 mg
- magnesium stearate: 10 mg
- talc: 50 mg
- aspartame: 15 mg
- metolose: 70 mg
- Arome: 20 mg
- sorbitol: 810 mg

6. Tablet including the composition according to claim 1 or claim 2, **characterized in that** it comprises the following formulation:
- ketoprofen lysinate: 5 mg
- magnesium stearate: 10 mg
- talc: 50 mg
- aspartame: 15 mg
- metolose: 70 mg
- Arome: 20 mg
- sorbitol: 830 mg

7. Use of the composition according to any of claims 1 to 4 for the production of a medication designed to locally treat buccopharyngeal ailments.
